# EUROPÄISCHE PATENTSCHRIFT

(11) **EP 1 707 543 B1**
(45) Veröffentlichungstag und Bekanntmachung des Hinweises auf die Patenterteilung: **14.11.2012**
(21) Anmeldenummer: 06011541.7
(22) Anmeldetag: 03.07.2001
(51) Int. Cl.: C04B 28/34, A61L 24/02, A61K 6/033

(54) **Magnesium-Ammonium-Phosphat-Zemente, deren Herstellung und Verwendung**
Magnesium-ammonium-phosphate cements, the production of the same and the use thereof
Ciments phosphate de magnesium-ammonium, leur preparation et leur utilisation

(30) Priorität: 03.07.2000 DE 10032220
(43) Veröffentlichungstag der Anmeldung: 04.10.2006
(62) Teilanmeldung aus: 01957914.3
(73) Patentinhaber: Sanatis GmbH, 01454 Radeberg (DE)
(72) Erfinder: Zimmermann, Michael, 65931 Frankfurt (DE)
(74) Vertreter: O'Connell, Maura

(56) Entgegenhaltungen:
- EP-A- 0 543 765
- WO-A-95/13835
- US-A- 4 612 053
- DATABASE CA [Online] CHEMICAL ABSTRACTS SERVICE, COLUMBUS, OHIO, US; PARK, SANG-JONG ET AL: "Compositional effects of CaO-SiO2-P2O5 bioactive cement on hardening and hydroxyapatite formation" XP002180738 gefunden im STN Database accession no. 121:141597 CA & YOOP HAKHOECHI (1994), 31(5), 502-12, 1994,
- DATABASE WPI Section Ch, Week 199204 Derwent Publications Ltd., London, GB; Class D22, AN 1992-027364 XP002180739 & JP 03 272771 A (NIPPON ELECTRIC GLASS CO) 4. Dezember 1991 (1991-12-04)

## Beschreibung

Die Erfindung betrifft eine Magnesiumammoniumphosphat-Zementzubereitung ein Verfahren zu deren Herstellung und eine zugehörige Verwendung.

Diese Erfindung betrifft insbesondere einen biologisch abbaubaren Zement, der nach Aushärtung *in seiner Hauptphase* aus Magnesiumammoniumphosphaten und Nanoapatiten besteht und dabei gleichzeitig eine hohe Festigkeit aufweist.

Das Material kann als Knochenersatz, zur Knochenaugmentation und zur Knochenregeneration eingesetzt werden.

Es kann als Trägermaterial für pharmazeutische oder biologische Wirkstoffe dienen

Die wichtigsten Mineralbestandteile im menschlichen Knochen und Zahnhartgewebe sind Calcium und Phosphat. Aber auch beachtliche Mengen von Natrium, Magnesium und Carbonat sind vorhanden.

Aus Präzipitationsstudien von synthetischen Systemen weiß man, daß Natriumionen und Carbonationen sehr leicht in Calciumphosphat-Präzipitate inkorporiert werden können mit dem Ergebnis einer apatitähnlichen Molekülstruktur.

Allerdings hat Magnesium eine starke Neigung zur Fällung in einer anderen, nicht apatitähnlichen Struktur.

Physiologisch als Knochen und Dentin präzipitiertes Calciumphosphat ist nanokristallin. Im Röntgendiffraktogramm ist wegen der Linienverbreiterungen nicht erkennbar, ob es sich dabei um Apatit oder um andere Strukturen handelt.

Einige Wissenschaftler sind der Meinung, daß in Knochen und Dentin so viel Magnesium vorkommt, daß dies nicht alles in der Apatitstruktur aufgenommen werden kann. Deshalb vermutet man hier eine Mischform des Minerals aus Nanoapatit und Nanodolomit bzw. Nanostruvit.

Calciumphosphate sind nicht nur biokompatibel sondern werden von der lebenden Zelle als körperzugehörig erkannt. Deshalb gibt es viele Biomaterialien und Medizinprodukte die teilweise aus Calciumphosphat bestehen.

Seit ca. 1970 gibt es Calciumphosphat - Keramiken auf dem Markt, teils in Form von vorgefertigten Blocks oder als Granulate.

Implantationen von diesen Materialien in Knochenstrukturen sind überwiegend erfolgreich.

Der größte Nachteil dieser Systeme ist, daß die Blocks vorgefertigt sein müssen und die Granulate von der Implantationsseite wegdriften (Ausschwemmen). Dies führt oft zum Scheitern solcher Implantationen.

Calciumphosphat - Keramiken sind höchst erfolgreich wenn sie aus Hydroxylapatit (HA) oder aus beta-tertiären Calciumphosphat (β-TCP, eine Whitlockit artige Struktur) bestehen oder wenn die Calciumphosphat - Keramiken aus beiden, HA und β-TCP in variablen Verhältnissen bestehen.

Aus Knochenimplantationen ist HA praktisch nicht resorbierbar wohingegen β-TCP langsam resorbiert und durch neuen Knochen ersetzt wird.

Es ist daher möglich, durch Verändern des β-TCP/HA - Verhältnisses den Grad der Resorption der Calciumphosphat - Keramik zu beeinflussen.

Es ist ebenso möglich, andere resorbierbare Stoffe beizumischen wie: Monetit CaHPO₄, Brushit CaHPO₄-2H₂O, Calcit CaCO₃ und Dolomit CaMg(CO₃)₂.

Seit 1985 versucht man Calciumphosphat-Zemente zu entwickeln um die Nachteile von vorgefertigten oder granulatförmigen Calciumphosphat-Keramiken zu umgehen (W.E. Brown and L.C. Chow, "A new calcium phosphate, water - setting cement", Cem. Res. Prog. 1986 352-379(1987)).

Darunter ist ein noch nicht im Handel befindlicher Brushit- Zement mit einem Ca/P Molverhältnis der gefällten Phase von 1,00. Diese Phase ist nicht nanokristallin sondem mikrokristallin.

Alle anderen bisher entwickelten Calciumphosphat-Zemente besitzen eine nanokristalline Fällungsstruktur und ein Ca/P Molverhältnis von >= 1,5 das durch Zusatz von Carbonat noch vergrößert werden kann. Diese Materialien sind unter U.S. 5,605,713; EP 0 835 668 A1; WO 96/14265; bekannt, und manche dieser Materialien sind bereits auf dem Markt.

Bezüglich der Resorbierbarkeit dieser Materialien nach Implantationen in Knochen und weichem Gewebe gibt es widersprüchliche Berichte.

In jedem Fall unterscheidet man Calciumphosphat-Zemente aufbauend auf Hydroxylapatit (HA) die nicht resorbierbar sind (HA Keramiken s.o.) und Calciumphosphat-Zemente aufbauend auf defiziente Calciumhydroxylapatiten (CDHA, Calcium deficient Hydroxyapatite) die gut osteotransductiv sind.

Das bedeutet für die Letztgenannten , daß sie von Osteoklasten resorbiert und von Osteoplasten durch neues Knochengewebe ersetzt werden können.

Die Resorption dieser Zemente hängt entscheidend von den lokalen Knochenumbaumechanismen ab.

Heutzutage wird von den meisten Chirurgen ein Calciumphosphat-Zement gewünscht, bei dem zunächst eine mechanisch unterstützende Wirkweise zum tragen kommt, aber die letztendliche Resorption unabhängig von den lokalen Umbaumechanismen des Knochens abhängt, d. h. daß das Material vollständig abgebaut wird. Außerdem ist in der Orthopädie bekannt daß vitaler Knochen nur dort verbleibt wo er aus biomechanischer Sicht benötigt wird. Dies ist als sogenanntes Wolffsches Gesetz bekannt. Hat demzufolge ein in einen Kochendefekt eingebrachter Calciumphosphat-Zement eine höhere Druckfestigkeit als der ihn umgebende Knochen und bleibt diese hohe Druckfestigkeit unverändert erhalten, so führt dies zum Abbau von um das Implantat (hier Calciumphosphat-Zement) liegende Knochengewebe.

Um diese Forderung wenn auch nur teilweise zu erfüllen, haben manche Hersteller Substanzen in ihre nanoapatit ähnlichen, CDHA-Zemente beigemischt die von den Körperflüssigkeiten aufgrund der Konzentrationsgradienten passiv resorbiert werden wie z.B. Monetit (CaHPO₄) oder Calcit (CaCO₃) wie aus EP 0 543 765 bekannt.

Dies löst das Problem aber nur teilweise. Es wird weiterhin ein Zement benötigt, der vollständig passiv resorbierbar ist und bei dem die Resorptionsfront und die Ablagerungsfront in direktem Kontakt liegen.

Gips beispielsweise erfüllt diese Forderung nicht. Gips ist so schnell resorbiert, daß immer eine Lücke zwischen der Resorptionsfront und der Ablagerungsfront klafft und diese Materialien aufgrund ihrer niedrigen Druckstabilität keine ausreichende unterstützende Funktion besitzen. Solche Materialien sind beispielsweise unter U.S. 5,281,265 offenbart.

Es ist aus diesen Gründen wünschenswert ein Knochenersatzmaterial zur Verfügung zu stellen, welches zunächst mit hoher Druckstabilität die verlorengegangene Stützfunktion des Knochens übernimmt, dann aber sukzessive an Druckstabilität abnimmt wodurch die körpereigenen Knochenumbauvorgänge (Remodelling) angeregt werden und somit eine schnellere Osteoneogenese und damit auch aktive Resorption des Knochenersatzmaterials eingeleitet wird. Dies kann auch dadurch erreicht werden, indem z.B. in eine aushärtende Zementpaste eine leicht lösliche Substanz eingearbeitet wird. Weil Knochen gut in makroporöse Strukturen einwächst ist es vorteilhaft, granuläre oder pelletförmige, solubilisierende Substanzen bestehend aus z.B. Zuckern, Salzen (z.B. NaCl) oder Gips (CaSO₄) in die Zementpaste einzumischen. Diese werden dann sehr schnell im Körper aus dem gehärteten Zementgerüst herausgelöst und übrig bleibt eine poröse schwammartige Struktur. Denkbar ist auch eine Herstellung der porösen (fertigen) Zemente außerhalb des Körpers.

Um einen Zement für dentale Anwendungen, wie z. B. Füllung und Verschluß von Dentinkanälchen, Füllung von Zahnhöhlen nach Vitalextirpation, Benutzung eines solchen Zementes als Unterfüllungswerkstoff in der Endodontologie, benutzen zu können, darf ein solches Material nicht schrumpfen um einen Durchtritt von Bakterien zu verhindern. Wünschenswert wäre sogar ein Material mit geringgradig expandierbaren Eigenschaften.

Es ist Aufgabe der Erfindung eine Zementzubereitung zur Verfügung zu stellen, mit der die Nachteile des Standes der Technik vermieden werden.

Die Aufgabe wird gelöst durch eine Zement-Zubereitung nach den Anspruch 1, , durch ein Verfahren zu deren Herstellung nach Anspruch 12, und durch die Verwendung nach den Ansprüchen 13 bis 15, sowie durch den Zement in Anspruch 16. Weiterbildungen der Erfindung sind in den Unteransprüchen angegeben.

Die Probleme beim Stand der Technik werden durch vorliegende Erfindung bevorzugt dahingehend gelöst, indem durch Variation in der Zumischung von Strontiumsalzen die Expansionsfähigkeit der aushärtenden Zementpaste eingestellt werden kann. In Versuchen, wie in den Beispielen dargelegt, zeigt sich nämlich, daß die Expansionsfähigkeit der Zementmischung, deren Hauptphase im ausgehärteten Zustand das Magnesiumammoniumphosphat darstellt, mit zunehmendem Gewichtsanteil von Strontiumsalzen an der Gesamtpulvermischung abnimmt. Demzufolge kann mit dieser Erfindung ein Werkstoff für die Endodontologie zur Verfügung gestellt werden der neben ausreichender mechanisch belastbaren Stabilität auch eine expandierbare Eigenschaft aufweist.
- Gegenstand dieser Erfindung ist es, ein Material für den Knochenersatz, für die Knochenaugmentation und die Knochenregeneration zur Verfügung zu stellen welches in einer begrenzten Zeit resorbiert werden kann und dessen Druckstabilität den Regenerationserfordernissen des Körpers angepaßt abnehmen kann
- Ebenso ist es Gegenstand dieser Erfindung ein Material zur Verfügung zu stellen, daß bei normalen Temperaturbedingungen, vorzugsweise Körpertemperatur, erstellt, vorbereitet und modelliert werden kann, in anderen Worten ein Zement.

Charakteristisch für das zur Verfügung gestellte Material ist, dass es zusätzlich durch die Stärke des Sinterungsgrades des eingebrachten Mg₃(PO₄)₂ in seiner Verarbeitungszeit, insbesondere bei Raumtemperatur, eingestellt werden kann, wobei durch den Sinterungsgrad und die daraus resultierende Dichte des verwendeten Mg₃(PO₄)₂ die Lösungsgeschwindigkeit an der Oberfläche dieser Partikel kontrolliert wird, sodaß die zur Erstarrung notwendige Präzipitation der ausfallenden Ca/Mg/Phosphat-Verbindung gesteuert werden kann.
- Weiterhin ist es Gegenstand dieser Erfindung einen Phosphatzement mit partieller Löslichkeit zur Verfügung zu stellen, vorzugsweise durch die langsame Löslichkeit der Magnesiumammoniumphosphat Apatitstruktur (Zement).
- Weiterhin ist Gegenstand der vorliegenden Erfindung einen Reaktionsprozess zu beschreiben, der aus einer Anzahl von Einzelkomponentenzur Formung eines Magnesiumammoniumphosphat-Zement führt und welcher in klinisch akzeptabler Zeit bei Raum- und/oder Körpertemperatur aushärtet.
- Weiterhin ist Gegenstand der vorliegenden Erfindung ein Material zur Verfügung zu stellen, welches in klinisch akzeptabler Zeit ausreichend hart und stabil wird und welches eine starke Adhäsionsfähigkeit an mineralisierten Oberflächen aufweist.
- Weiterhin ist Gegenstand der Erfindung sich das nach Art der Erfindung offenbarte Material durch eine starke Adhäsionsfähigkeit an metallischen Oberflächen auszeichnet.
- Weiterhin ist Gegenstand der vorliegenden Erfindung einen resorbierbaren Zement zur Verfügung zu stellen der im Stadium einer gemischten Paste injizierbar ist.
- Ein Aspekt dieser Erfindung ist, dass das Endprodukt aus einer Pulvermischung besteht mit einem molaren Ca/P Verhältnis im Bereich von 1,00 bis 1,50. (P steht für Orthophosphat).
- Außerdem ist es essentiell, dass das molare Mg/P Verhältnis dieser Pulvermischung den Bereich von 0 bis 1,00 umfasst.
- Um eine Zementpaste zu mischen und zu formen, die innerhalb einer akzeptablen Zeit härtet, müssen diese Pulvermischungen ausreichend reaktiv sein. Um dies zu erreichen ist ein weiterer Aspekt dieser Erfindung die Pulvermischungen mit geeigneten Mengen leicht basischer (7<pH<12), wässriger Lösungen aus löslichen ionischen Bestandteilen zu mischen wie beispielsweise: Na₃PO₄, K₂CO₃, und/oder Na₂CO₃ in Kombination mit (NH₄)₂HPO₄.
- Ein weiteres Merkmal dieser Erfindung ist, dass der aushärtenden Zementpaste granuläre aber dabei in der Körperflüssigkeit leicht lösliche , granuläre Feststoffe zugemischt werden, so dass nach deren Herauslösung ein mikro- bis makroporöses Porensystem entsteht.
- Ein anderer Aspekt dieser Erfindung ist, dass diese Zemente ihre maximale Festigkeit innerhalb weniger Stunden erreichen.
- Ein weiteres Merkmal dieser Erfindung liegt in der Expansionsfähigkeit des Zementes während des Abbindens. Die Expansivität wird durch den relativen Anteil eines zugemischten Strontiumsalzes bestimmt bzw. eingestellt.
- Ein weiteres Merkmal dieser Erfindung ist, dass der ausgehärtete Zement aus mikrokristallinem Magnesiumammoniumphosphat besteht.
- Ein weiteres Merkmal dieser Erfindung ist, dass die initiale Aushärtezeit des Zementes auf 1 bis 40 Minuten und die finale Aushärtezeit auf 2,5 bis 60 Minuten eingestellt werden kann. (nach ASTM C266-89)
- Ein weiteres Merkmal dieser Erfindung ist, dass der Zement eine maximale Kompressionsfestigkeit von über 50 MPa erreichen kann
- Ein weiteres Merkmal dieser Erfindung ist, dass die Zementpaste vor Erreichen der initialen Aushärtezeit injizierbar ist.
- Ein weiteres Merkmal dieser Erfindung ist, dass die Zementpaste als Trägermaterial für andere Stoffe die nicht Ca, Mg und/oder Phosphat sind, dienen kann. Z.B. ZnO, pharmazeutische Wirkstoffe (Antibiotika, Zytostatika, Wachstumsfaktoren) oder andere bioaktive Substanzen.

Weitere Merkmale und Zweckmäßigkeiten der Erfindung ergeben sich aus der Beschreibung von Ausführungsbeispielen.

### Beispiele:

In den Beispielen werden folgende Symbole benutzt:
- P =: Pulvermischung
- L =: Flüssigkeit
- L/P =: Flüssigkeit/Pulver Verhältnis in ml/g
- tₗ =: initiale Aushärtezeit (nach ASTM-Norm C266-89, Gilmoore Nadel)
- t_{F} =: finale (end-) Aushärtezeit (nach ASTM-Norm C266-89 Gilmoore Nadel)
- D(x h) =: Kompressionsfestigkeit in Mpa nach x-Stunden Lagerung in 0,9%iger NaCl Lösung bei 37°C

### Herstellung: Nach Einwiegen aller Bestandteile wird die Pulvermischung in einer Kugelmühle für ca. 20 Minuten homogenisiert.

### Vergleichsbeispiel 1:

| | | | | | | |
|---|---|---|---|---|---|---|
| P= | 60g α-Ca₃(PO₄)₂ + 6g MgHPO₄ • 3H₂O + 5g MgSO₄ • 7H₂O | | | | | |
| L= | 2 M (NH₄)₂HPO₄ | | | | L/P = | 0,40 |
| tₗ= | 9 | T_{F} = | 21 | | | |
| D(18) | 18,4 ± 1,5 | | | | | |
| D(72) | 26,1 ± 4,0 | | | | | |

### Vergleischsbeispiel 2:

| | | | | | | |
|---|---|---|---|---|---|---|
| P = | 60g α-Ca₃(PO₄)₂ + 14g MgHPO₄ • 3H₂O + 2g Mg(OH)₂ | | | | | |
| L = | 3,5 M (NH₄)₂HPO₄ | | | | L/P = | 0,35 |
| tₗ = | 3 | T_{F} = | 7 | | | |
| D(18) | 32,5 ± 3,5 | | | | | |
| D(72) | 46,9 ± 5,4 | | | | | |

### Vergleichsbeispiel 3:

| | | | | | | |
|---|---|---|---|---|---|---|
| P = | 60g α-Ca₃(PO₄)₂ + 16g MgHPO4·3H₂O + 3g Na₃(PO₄)₃ • 12H₂O | | | | | |
| L = | 3,5 M (NH₄)₂PO₄ | | | | L/P = | 0,35 |
| tₗ = | 6 | T_{F} = | 14 | | | |
| D(18) | 44,7 ± 3,4 | | | | | |
| D(72) | 51,7 ± 5,0 | | | | | |

### Vergleichsbeispiel 4:

| | | | | | | |
|---|---|---|---|---|---|---|
| P = | 60g α-Ca₃(PO₄)₂ + 14g MgHPO₄ • 3H₂O + 2g ZnO | | | | | |
| L = | 3,5 M (NF₄)₂HPO₄ | | | | L/P = | 0,35 |
| tₗ = | 6 | T_{F} = | 23 | | | |
| D(16) | 35,1 ± 5,3 | | | | | |
| D(72) | 42,9 ± 0,8 | | | | | |

### Vergleichsbeispiel 5:

| | | | | | | |
|---|---|---|---|---|---|---|
| P= | 45g CaHPO₄•2 H₂O + 14g CaCO₃ + 14g MgHPO₄•3H₂O + 6g Mg(OH)₂ | | | | | |
| L= | 2 M (NH₄)₂HPO₄ | | | | L/P = | 0,40 |
| tₗ = | 2,5 | T_{F} = | 7,5 | | | |
| D(18) | 3,8 ± 1,2 | | | | | |

### Vergleichsbeispiel 6:

| | | | | | | |
|---|---|---|---|---|---|---|
| P = | 45g CaHPO₄•2 H₂O + 14g CaCO₃ + 14g MgHPO₄•3H₂O + 6g ZnO | | | | | |
| L = | 3,5 M (NH₄)₂HPO₄ | | | | L/P = | 0,35 |
| tₗ= | 2 | T_{F} = | 4 | | | |
| D(18) | 3,8 ± 1,2 | | | | | |

### Vergleichsbeispiel 7:

| | | | | | | |
|---|---|---|---|---|---|---|
| P = | 60g α-Ca₃(PO₄)₂ + 16g MgHPO₄ • 3H₂O + 5g β-Ca₃(PO₄)₂ | | | | | |
| L = | 3,5 M (NH₄)₂HPO₄ | | | | L/P = | 0,35 |
| tₗ = | 4 | T_{F} = | 9 | | | |
| D(2) | 59,3 ± 1,0 | | | | | |
| D(4) | 55,6 ± 5,0 | | | | | |
| D(18) | 61,6 ± 5,0 | | | | | |
| D(72) | 51,5 ± 6,6 | | | | | |
| D(18d) | 28,1 ± 4,6 | | | | | |

### Vergleichsbeispiel 8:

| | | | | | | |
|---|---|---|---|---|---|---|
| P = | 60g α-Ca₃(PO₄)₂ + 16g MgHPO₄ • 3H₂O + 5g β-Ca₃(PO₄)₂ | | | | | |
| L= | 3,5 M (NH₄)₂HPO₄ | | | | L/P = | 0,35 |
| tₗ = | 3,5 | T_{F} = | 11, 5 | | | |
| D(2,5) | 54,4 ± 3,3 | | | | | |
| D(18) | 65,6 ± 5,3 | | | | | |
| D(4d) | 56,6 ± 8,6 | | | | | |
| D(18d) | 36,3 ± 2,4 | | | | | |
| D(30d) | 30,0 ± 3,0 | | | | | |

### Beispiel 9:

| | | | | | | |
|---|---|---|---|---|---|---|
| P = | 60g α-Ca₃(PO₄)₂ + 16g MgHPO₄•3H₂O + 5g β-Ca₃(PO₄)₂ + 0,8g SrCO₃ | | | | | |
| L= | 3,5 M (NH₄)₂HPO₄ | | | | L/P = | 0,35 |
| tₗ = | 5,5 | T_{F} = | 13 | | | |
| D(2,5) | 54,3 ± 4,6 | | | | | |
| D(5) | 61,1 ± 5,5 | | | | | |
| D(18) | 70,1 ± 5,7 | | | | | |
| D(4d) | 74,3 ± 9,3 | | | | | |
| D(18d) | 43,4 ± 3,4 | | | | | |
| D(30d) | 34,0 ± 4,0 | | | | | |

### Beispiel 10:

| | | | | | | |
|---|---|---|---|---|---|---|
| P = | 60g α-Ca₃(PO₄)₂ + 8g MgHPO₄ • 3H₂O + 2g (NH₄)₂SO₄ + 2g KH₂PO₄ + 3,5g SrCO₃ | | | | | |
| L= | 3,5 M (NH₄)₂HPO₄ | | | | L/P = | 0,30 |
| tₗ = | | T_{F} = | | | | |
| D(0,25) | 11,2 ± 0,8 | | | | | |
| D(0,5) | 17,2 ± 1,8 | | | | | |
| D(2) | 31,7 ± 1,3 | | | | | |
| D(6) | 39,7 ± 0,63 | | | | | |
| D(3d) | 56,5 ± 4,9 | | | | | |

### Beispiel 11:

| | | | | | | |
|---|---|---|---|---|---|---|
| P = | 60g α-Ca₃(PO₄)₂ + 8g MgHPO₄ • 3H₂O + 4g (NH₄)H₂PO₄ + 1g SrCO₃ | | | | | |
| L= | 3,5 M (NH₄)₂HPO₄ | | | | L/P = | 0,37 |
| tₗ = | | T_{F} = | | | | |
| D(2) | 22,6 ± 1,0 | | | | | |
| D(6) | 31,4 ± 1,1 | | | | | |
| D(18) | 45,8 ± 1,8 | | | | | |
| D(3d) | 45,7 ± 2,9 | | | | | |
| D(35d) | 11,5 ± 1,2 | | | | | |

### Beispiel 12:

| | | | | | | |
|---|---|---|---|---|---|---|
| P = | 60g α-Ca₃(PO₄)₂ + 17,4g MgHPO₄ • 3H₂O + 7g (NH₄)₂SO₄ + 1,7g SrCO₃ | | | | | |
| L = | 3,5 M (NH₄)₂HPO₄ | | | | L/P = | 0,35 |
| tₗ = | | T_{F} = | | | | |
| D(2) | 43,3 ± 2,9 | | | | | |
| D(6) | 45,4 ± 4,4 | | | | | |
| D(18) | 45,8 ± 1,8 | | | | | |
| D(3d) | 45,7 ± 2,9 | | | | | |
| D(28d) | 19,5 ± 5,1 | | | | | |

### Beispiel 13:

| | | | | | | |
|---|---|---|---|---|---|---|
| P = | 60g α-Ca₃(PO₄)₂ + 20g CaHPO₄ + 8g CaCO₃ + 1g MgHPO₄ + 1,7g SrCO₃ | | | | | |
| L= | 3,5 M (NH₄)₂HPO₄ | | | | L/P = | 0,35 |
| tₗ =2,5 | | T_{F}=8 | | | | |
| D(2) | 43,3 ± 2,9 | | | | | |
| D(6) | 49,4 ± 3,7 | | | | | |
| D(18) | 54,3 ± 2,5 | | | | | |
| D(3d) | 53,6 ± 3,1 | | | | | |
| D(28d) | 54,5 ± 1,9 | | | | | |

### Beispiel 14:

| | | | | | | |
|---|---|---|---|---|---|---|
| P = | 60g β-Ca₃(PO₄)₂ + 17,4g MgHPO₄ • 3H₂O +1,7g SrCO₃ | | | | | |
| L = | 3,5 M (NH₄)₂HPO₄ | | | | L/P = | 0,35 |
| tₗ =3,5 | | T_{F}=9 | | | | |

### Vergleichsbeispiel 15:

| | | | | | | |
|---|---|---|---|---|---|---|
| P = | 65g α-TCP + 34,8g KgHPO₄x3H₂O +13,2g (NH₄)₂SO₄ | | | | | |
| L= | 5% NaHCO₃ | | | | L/P = | 0,35 |
| tₗ =3 | | T_{F}=10 | | | | |

### Vergleichsbeispiel 16:

| | | | | | | |
|---|---|---|---|---|---|---|
| P = | 60g α-TCP + 16g MgHPO₄x3H₂O +5g β-TCP + 20g NaCl | | | | | |
| | | | | | (Durchmesser 150 µm) | |
| L = | 3,5M (NH₄)₂HPO₄ | | | | L/P = | 0,35 |
| tₗ = 5 | | T_{F}=12 | | | | |
| | | | | | | |

### Vergleichsbeispiel 17:

P= 60g α-TCP + 6g Mg₃(PO₄)₂ + 10g KH₂PO₄ + 5g β-TCP Anmischlösung: 3,2 molare Lsg. (NH₄)₂HPO₄
UP = 0,35
Tᵢ= 10,8 tᵢ= 20
D(2) = 18,5 ± 1,0
D(18) = 48,3 ± 1,8

## Patentansprüche

1. Magnesium-ammonium-phosphat-Zement-Zubereitung, umfassend
- eine Pulvermischung mit Komponenten Calcium (Ca), Magnesium (Mg) und Orthophosphat (P) in der Mischung, wobei das molare Ca/P - Verhältnis den Bereich von 1,00 bis 1,50 umfasst und das molare Mg/P-Verhältnis den Bereich von 0 bis 1,00 umfasst,
- ein Strontiumsalz;
- ein Ammoniumsalz; und
- gegenbenfalls Wasser und /oder eine wässrige Lösung.

2. Zubereitung nach Anspruch 1, worin die wässrige Lösung eine wässrige Lösung eines Ammoniumsalzes mit einem pH-Wert im Bereich von 7 < pH < 12 ist.

3. Zubereitung nach Anspruch 1, worin das Ammoniumsalz in der Pulvermischung enthalten ist und die molaren Mengen der Komponenten Calcium (Ca), Magnesium (Mg), Orthophosphat (P) und Ammonium (NH₄) in den Bereichen 1,00 < Ca/P < 1,50 und 0 < Mg/P < 0,50 und 0 < NH₄ < 0,50 liegen.

4. Zubereitung nach einem der Ansprüche 1 bis 3, umfassend SrCO₃ als Strontiumsalz.

5. Zubereitung nach Anspruch 4, worin der Gehalt an SrCO₃ 0,01 bis 10 Gew.-% beträgt, vorzugsweise 0,1 bis 5 Gew.-%, bezogen auf das Gesamtgewicht der Zubereitung.

6. Zubereitung nach einem der Ansprüche 1 bis 5, umfassend als Strontiumsalz und/oder Magnesiumsalz eine wässrige Lösung jeweils eines Strontiumsalzes und / oder Magnesiumsalzes in Form einer Anmischflüssigkeit.

7. Zubereitung nach einem der Ansprüche 1 bis 6, umfassend als zusätzliche Komponente ZnO in der Pulvermischung und/oder in der Anmischflüssigkeit.

8. Zubereitung nach einem der Ansprüche 1 bis 7, umfassend als zusätzliche Komponente Fluorid-Ionen in der Pulvermischung und/oder in der Anmischflüssigkeit.

9. Zubereitung nach einem der Ansprüche 1 bis 8, wobei die Pulvermischung ferner granuläre Partikel umfaßt, die in wässrigen Flüssigkeiten leicht löslich sind und Durchmesser zwischen etwa 10µm und etwa 300µm, vorzugsweise zwischen etwa 50µm und etwa 200µm aufweisen.

10. Zubereitung gemäß einem der Ansprüche 1 bis 9, die eine initiale Aushärtezeit auf 1 bis 40 Minuten und eine finale Aushärtezeit von 2,5 bis 60 Minuten (nach ASTM C266-89) besitzt.

11. Zubereitung nach einem der Ansprüche 1 bis 10, umfassend als zusätzliche Komponenten pharmazeutische und/oder bioaktive Wirkstoffe in der Pulvermischung und/oder in der Anmischflüssigkeit, bevorzugt Antibiotika, Zytostatika, Analgetika, Desinfizienzien, Wachstumsfaktoren, Proteine oder Elastininhibitoren in therapeutischen Dosen.

12. Verfahren zur Herstellung eines Magnesium-ammonium-phosphat-Zements nach einem der Ansprüche 1 bis 11, worin man die Pulvermischung mit der Anmischflüssigkeit unter Erzielen einer gleichmäßigen Verteilung der Flüssigkeit in der Pulvermischung mischt und die so erhaltene Paste an bzw. auf der Zielzone aufträgt bzw. in die Zielzone einbringt und aushärten lässt, wobei die Komponenten derart reagieren, daß der gebildete Zement mikrokristallines Magnesiumammoniumphosphat aufweist.

13. Zubereitung nach einem der Ansprüche 1 bis 11 zur Verwendung für medizinische Zwecke.

14. Zubereitung nach einem der Ansprüche 1 bis 11 zur Verwendung als Zahnzement.

15. Zubereitung nach einem der Ansprüche 1 bis 11 zur Verwendung als Knochenersatz oder Knochenfüllstoff oder Knochenzement oder Knochenklebstoff.

16. Magnesiumammoniumphosphat-Zement, dessen Hauptphase aus Magnesiumammoniumphosphat nach Anspruch 1 und Nanoapatiten besteht, und das eine Kompressionsfestigkeit von über 50 MPa aufweist.

17. Magnesiumammoniumphosphat-Zement gemäß Anspruch 16, das expandierbar ist.

18. Magnesiumammoniumphosphat-Zement gemäß Anspruch 16 oder 17, erhalten durch Härten einer Magnesium-ammonium-phosphat-Zement-Zubereitung nach einem der Ansprüche 1 bis 11 oder durch das Verfahren nach Anspruch 12.

## Claims

1. Magnesium ammonium phosphate cement preparation comprising:
- a powder mixture having calcium (Ca), magnesium (Mg) and orthophosphate (P) components in the mixture, wherein the Ca/P molar ratio includes the range of 1.00 to 1.50 and the Mg/P molar ratio includes the range of 0 to 1.00,
- a strontium salt;
- an ammonium salt; and
- optionally water and/or an aqueous solution.

2. Preparation as claimed in claim 1, wherein the aqueous solution is an aqueous solution of an ammonium salt having a pH value in the range of 7 < pH < 12.

3. Preparation as claimed in claim 1, wherein the ammonium salt is contained in the powder mixture and the molar quantities of the calcium (Ca), magnesium (Mg), orthophosphate (P) and ammonium (NH₄) components are in the ranges 1.00 < Ca/P < 1.50 and 0 < Mg/P < 0.50 and 0 < NH4 < 0.50.

4. Preparation as claimed in any one of claims 1 to 3, comprising SrCO₃ as the strontium salt.

5. Preparation as claimed in claim 4, wherein the SrCO₃ content amounts to 0.01 to 10 wt. %, preferably 0.1 to 5 wt.% based on the total weight of the preparation.

6. Preparation as claimed in any one of claims 1 to 5, comprising as the strontium salt and/or magnesium salt an aqueous solution of a strontium salt and/or of a magnesium salt respectively in the form of a mixing liquid.

7. Preparation as claimed in any one of claims 1 to 6, comprising, as an additional component, ZnO in the powder mixture and/or in the mixing liquid.

8. Preparation as claimed in any one of claims 1 to 7, comprising, as an additional component, fluoride ions in the powder mixture and/or in the mixing liquid.

9. Preparation as claimed in any one of claims 1 to 8, wherein the powder mixture further comprises granular particles which are readily soluble in aqueous liquids and have diameters between about 10µm and about 300µm, preferably between about 50µm and about 200µm.

10. Preparation as claimed in any one of claims 1 to 9, which has an initial hardening time of 1 to 40 minutes and a final hardening time of 2.5 to 60 minutes (according to ASTM C266-89).

11. Preparation as claimed in any one of claims 1 to 10, comprising, as additional components, pharmaceutical and/or bioactive active ingredients in the powder mixture and/or in the mixing liquid, preferably antibiotics, cytostatic agents, analgesics, disinfectants, growth factors, proteins or elastin inhibitors in therapeutic doses.

12. Method for producing a magnesium ammonium phosphate cement as claimed in any one of claims 1 to 11, wherein the powder mixture is mixed with the mixing liquid while achieving a uniform distribution of the liquid in the powder mixture, and the paste thus obtained is applied at or on the target zone or is introduced into the target zone and allowed to harden, wherein the components react such that the formed cement has a microcrystalline magnesium ammonium phosphate.

13. Preparation as claimed in any one of claims 1 to 11 to be used for medical purposes.

14. Preparation as claimed in any one of claims 1 to 11 to be used as a tooth cement.

15. Preparation as claimed in any one of claims 1 to 11 to be used as a bone replacement or bone filler or bone cement or bone adhesive.

16. Magnesium ammonium phosphate cement, the main phase of which consists of magnesium ammonium phosphate as claimed in claim 1 and nano apatites and which has a compressive strength of more than 50 MPa.

17. Magnesium ammonium phosphate cement as claimed in claim 16, which is expandable.

18. Magnesium ammonium phosphate cement as claimed in claim 16 or 17, obtained by hardening a magnesium ammonium phosphate cement preparation as claimed in any one of claims 1 to 11 or by the method as claimed in claim 12.

## Revendications

1. Composition de ciment de phosphate de magnésium-ammonium, comprenant
- un mélange pulvérulent comportant les composants calcium (Ca), magnésium (Mg) et orthophosphate (P) dans le mélange, où le rapport molaire Ca-P se situe dans la plage de 1,00 à 1,50 et le rapport molaire Mg-P se situe dans la plage de 0 à 1,00,
- un sel de strontium,
- un sel d'ammonium et
- le cas échéant de l'eau et/ou une solution aqueuse.

2. Composition selon la revendication 1, où la solution aqueuse est une solution aqueuse d'un sel d'ammonium présentant un indice de pH dans la plage 7 < pH < 12.

3. Composition selon la revendication 1, où le sel d'ammonium est contenu dans le mélange pulvérulent et les valeurs molaires des composants calcium (Ca), magnésium (Mg), orthophosphate (P) et ammonium (NH₄) se situent dans les plages 1,00 < Ca-P < 1,50 et 0 < Mg-P < 0,50 et 0 < NH₄ < 0,50.

4. Composition selon l'une des revendications 1 à 3, comprenant du SrCO₃ en tant que sel de strontium.

5. Composition selon la revendication 4, où la teneur en SrCO₃ se situe dans la plage de 0,01 à 10 % en poids, de préférence de 0,1 à 5 % en poids, par rapport au poids total de la composition.

6. Composition selon l'une des revendications 1 à 5, comprenant en tant que sel de strontium et/ou sel de magnésium une solution aqueuse respectivement d'un sel de strontium et/ou d'un sel de magnésium sous forme d'un liquide d'adjonction.

7. Composition selon l'une des revendications 1 à 6, comprenant en tant que composant supplémentaire du ZnO dans le mélange pulvérulent et/ou dans le liquide d'adjonction.

8. Composition selon l'une des revendications 1 à 7, comprenant en tant que composants supplémentaires des ions fluorure dans le mélange pulvérulent et/ou le liquide d'adjonction.

9. Composition selon l'une des revendications 1 à 8, où le mélange pulvérulent comporte en outre des particules granulaires qui sont facilement solubles dans des liquides aqueux et présentent des diamètres situés entre environ 10µm et environ 300µm, de préférence entre environ 50µm et environ 200µm

10. Composition selon l'une des revendications 1 à 9, qui offre un temps de durcissement initial de 1 à 40 minutes et un temps de durcissement final de 2,5 à 60 minutes (conformément à ASTM C266-89).

11. Composition selon l'une des revendications 1 à 10, comprenant en tant que composants supplémentaires des principes actifs pharmaceutiques et/ou bioactifs dans le mélange pulvérulent et/ou le liquide d'adjonction, de préférence des antibiotiques, des cytostatiques, des analgésiques, des désinfectants, des facteurs de croissance, des protéines ou des inhibiteurs d'élastine en doses thérapeutiques.

12. Procédé de fabrication d'un ciment de phosphate de magnésium-ammonium selon l'une des revendications 1 à 11, où l'on mélange le mélange pulvérulent et le liquide d'adjonction en obtenant une répartition homogène du liquide dans le mélange pulvérulent et où la pâte ainsi obtenue est déposée sur ou éventuellement à la zone ciblée ou encore est introduite éventuellement dans la zone ciblée et où elle est laissée à durcir, les composants réagissant de telle sorte que le ciment ainsi constitué contient un phosphate de magnésium-ammonium microcristallin.

13. Composition selon l'une des revendications 1 à 11 pour utilisation à fins médicales.

14. Composition selon l'une des revendications 1 à 11 pour utilisation en tant que ciment dentaire.

15. Composition selon l'une des revendications 1 à 11, pour utilisation en tant qu'implant osseux ou charge osseuse ou en tant que ciment osseux ou colle osseuse.

16. Ciment de phosphate de magnésium-ammonium, dont la phase principale se compose de phosphate de magnésium-ammonium selon la revendication 1 et de nanoapatites, et qui présente une résistance à la compression supérieure à 50 MPa.

17. Ciment de phosphate de magnésium-ammonium selon la revendication 16 qui est expansible.

18. Ciment de phosphate de magnésium-ammonium selon la revendication 16 ou 17, obtenu par durcissement d'une composition de ciment de phosphate de magnésium-ammonium selon l'une des revendications 1 à 11 ou conformément au procédé selon la revendication 12.
